# EUROPÄISCHE PATENTSCHRIFT

(11) **EP 2 387 556 B1**
(45) Veröffentlichungstag und Bekanntmachung des Hinweises auf die Patenterteilung: **30.07.2014**
(21) Anmeldenummer: 10700413.7
(22) Anmeldetag: 15.01.2010
(51) Int. Cl.: C07C 213/10, C07C 215/08, C07C 217/10, C07C 59/50

(54) **TRENNUNG EINES ENANTIOMERENGEMISCHS VON (R)- UND (S)-3-AMINO-1-BUTANOL**
SEPARATION OF AN ENANTIOMER MIXTURE OF (R)- AND (S)-3-AMINO-1-BUTANOL
SÉPARATION D'UN MÉLANGE D'ÉNANTIOMÈRES DE (R)- ET DE (S)-3-AMINO-1-BUTANOL

(30) Priorität: 16.01.2009 EP 09150780
(43) Veröffentlichungstag der Anmeldung: 23.11.2011
(73) Patentinhaber: BASF SE, 67056 Ludwigshafen (DE)
(72) Erfinder: DITRICH, Klaus, 67161 Gönnheim (DE); BARTSCH, Michael, CH-8816 Hirzel (CH); WINSEL, Harald, 67251 Freinsheim (DE)
(86) Internationale Anmeldenummer: PCT/EP2010/050434
(87) Internationale Veröffentlichungsnummer: WO 2010/081865

(56) Entgegenhaltungen:
- EP-B1- 0 801 683
- WO-A2-01/38292
- US-A1- 2008 021 048
- BESSE P ET AL: "Stereoselective chemoenzymatic synthesis of both enantiomers of protected 4-amino-2-pentanone" TETRAHEDRON ASYMMETRY, PERGAMON PRESS LTD, OXFORD, GB, Bd. 10, Nr. 11, 4. Juni 1999 (1999-06-04), Seiten 2213-2224, XP004174104 ISSN: 0957-4166

## Beschreibung

Die vorliegende Erfindung betrifft ein Verfahren zur Trennung eines Enantiomerengemischs von gegebenenfalls am Sauerstoffatom geschütztem (R)- und (S)-3-Amino-1-butanol sowie ein Verfahren zur Herstellung von im Wesentlichen enantiomerenreinem (R)-3-Amino-1-butanol, das gegebenenfalls am Sauerstoffatom eine Schutzgruppe trägt.

Optisch aktive Verbindungen besitzen vor allem im Bereich der pharmazeutischen Industrie immense Bedeutung, da häufig nur ein bestimmtes optisch aktives Isomer therapeutisch aktiv ist. Somit besteht ein stetig wachsender Bedarf an optisch aktiven Verbindungen als Edukte für die enantioselektive Synthese von Wirkstoffen. Einer dieser Schlüsselbausteine für die Synthese eines Mittels zur Behandlung von AIDS-Symptomen ist das optisch aktive 3-Amino-1-butanol und insbesondere dessen (R)-Enantiomer.

Versuche der Anmelderin, racemisches, am Sauerstoffatom Benzyl-geschütztes 3-Amino-1-butanol über den Weg der enzymkatalysierten selektiven N-Acylierung und anschließenden Hydrolyse in seine Enantiomere zu spalten, führte zwar zum gewünschten (R)-Enantiomer in befriedigender Enantiomerenreinheit, jedoch nur über aufwändige und zahlreiche Verfahrensschritte und einer nicht zufriedenstellenden Gesamtausbeute.

WO 01/38292 betrifft ein Verfahren zur Herstellung optisch aktiver Amine. Die Amine enthalten neben der Aminogruppe noch eine weitere Aminogruppe oder eine Ethergruppe. Das Verfahren umfasst unter anderem einen Racematspaltungsschritt, bei dem das racemische Amin mit einer optisch aktiven Carbonsäure oder einem Ester davon in Gegenwart einer Lipase oder Esterase umgesetzt wird, wobei ein Gemisch aus einem acylierten Amin-Enantiomer und seinem nicht acylierten Antipoden erhalten wird. Das Gemisch wird getrennt und das acylierte Enantiomer wird gewünschtenfalls freigesetzt.

Aufgabe der vorliegenden Erfindung war es daher, ein effizienteres Verfahren zur Her-stellung von im Wesentlichen enantiomerenreinem (S)- und insbesondere (R)-3-Amino-1-butanol zur Verfügung zu stellen, welches in wenigen, einfachen Schritten und mit befriedigender Ausbeute durchzuführen ist. Insbesondere sollte das Verfahren die Her-stellung von (R)-3-Amino-1-butanol in einer Enantiomerenreinhelt von wenigstens 98 % ee, vorzugsweise wenigstens 99 % ee, besonders bevorzugt wenigstens 99,5 % ee, stärker bevorzugt wenigstens 99,6 % ee und insbesondere wenigstens 99,8 % ee erlauben.

Überraschenderweise wurde festgestellt, dass die Verwendung von im Wesentlichen enantiomerenreiner Mandelsäure, also von (S)- oder (R)-Mandelsäure, die Herstellung von (R)-3-Amino-1-butanol und gewünschtenfalls auch von (S)-3-Amino-1-butanol und auch der entsprechenden am Sauerstoffatom geschützten Verbindungen in hohen Enantiomerenreinheiten und auch zufriedenstellenden Ausbeuten erlaubt.

Gegenstand der Erfindung ist daher ein Verfahren zur Trennung eines Enantiomerengemischs von gegebenenfalls am Sauerstoffatom geschütztem (R)- und (S)-3-Amino-1-butanol, umfassend folgende Schritte:
(i) Umsetzen eines Enantiomerengemischs der Verbindungen der Formeln I-R und I-S 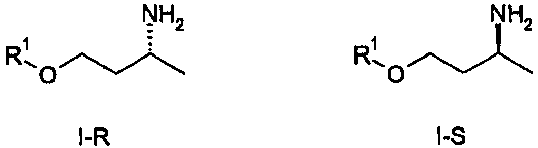
   worin R¹ für Wasserstoff oder eine Schutzgruppe die unter C₁-C₄-Alkyl ausgewählt ist, steht,
   mit (S)- oder (R)-Mandelsäure und einer von dieser eingesetzten (S)- oder (R)-Mandelsäure verschiedenen Säure;
(ii) Abtrennen und Isolieren des in Schritt (i) gebildeten (S)- oder (R)-Mandelsäuresalzes der Verbindung der Formel I-R;
(iii) gegebenenfalls Reinigen des in Schritt (ii) isolierten (S)- oder (R)-Mandelsäuresalzes der Verbindung der Formel I-R;
(iv) Freisetzen der Verbindung der Formel I-R aus ihrem (S)- oder (R)-Mandelsäuresalz und gewünschtenfalls Entschützen der Verbindung der Formel I-R, worin R¹ von Wasserstoff verschieden ist, unter Erhalt von (R)-3-Amino-1-butanol; und
(v) gewünschtenfalls Freisetzen der (angereicherten) Verbindung der Formel I-S und gewünschtenfalls Entschützen der Verbindung der Formel I-S, worin R¹ von Wasserstoff verschieden ist, unter Erhalt von (angereichertem) (S)-3-Amino-1-butanol,
wobei das Freisetzen der Verbindung der Formel I-R aus ihrem (S)- oder (R)-Mandelsäuresalz in Schritt (iv) durch Umsetzung des in Schritt (ii) oder (iii) erhaltenen (S)-oder (R)-Mandelsäuresalzes der Verbindung der Formel I-R mit einer Base, die unter Alkalimetall-C₁-C₄-alkoxiden ausgewählt ist, in einem Lösungsmittel, das einen um wenigstens 10 °C höheren Siedepunkt als 3-Amino-1-butanol besitzt und unter organischen Aminen mit einem Schmelzpunkt von höchstens 50 °C und einem Siedepunkt von wenigstens 180 °C ausgewählt ist, erfolgt.

Gegenstand der Erfindung ist außerdem ein Verfahren zur Herstellung von im Wesentlichen enantiomerenreinem (R)-3-Amino-1-butanol (d.h. mit einer Enantiomerenreinheit von wenigstens 95%ee), das gegebenenfalls am Sauerstoffatom eine Schutzgruppe trägt, umfassend folgende Schritte:
(i) Umsetzen eines Enantiomerengemischs der Verbindungen der Formeln I-R und I-S 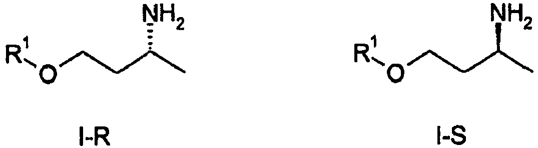
   worin R¹ für Wasserstoff oder eine Schutzgruppe, die unter C₁-C₄-Alkyl ausgewählt ist, steht,
   mit (S)- oder (R)-Mandelsäure und einer von dieser eingesetzten (S)- oder (R)-Mandelsäure verschiedenen Säure;
(ii) Abtrennen und Isolieren des in Schritt (i) gebildeten (S)- oder (R)-Mandelsäuresalzes der Verbindung der Formel I-R;
(iii) gegebenenfalls Reinigen des in Schritt (ii) isolierten (S)- oder (R)-Mandelsäuresalzes der Verbindung der Formel I-R; und
(iv) Freisetzen der Verbindung der Formel I-R aus ihrem (S)- oder (R)-Mandelsäuresalz und gewünschtenfalls Entschützen der Verbindung der Formel I-R, worin R¹ von Wasserstoff verschieden ist, unter Erhalt von (R)-3-Amino-1-butanol,
wobei das Freisetzen der Verbindung der Formel I-R aus ihrem (S)- oder (R)-Mandelsäuresalz in Schritt (iv) durch Umsetzung des in Schritt (ii) oder (iii) erhaltenen (S)-oder (R)-Mandelsäuresalzes der Verbindung der Formel I-R mit einer Base, die unter Alkalimetall-C₁-C₄-alkoxiden ausgewählt ist, in einem Lösungsmittel, das einen um wenigstens 10 °C höheren Siedepunkt als 3-Amino-1-butanol besitzt und unter organischen Aminen mit einem Schmelzpunkt von höchstens 50 °C und einem Siedepunkt von wenigstens 180 °C ausgewählt ist, erfolgt.

Der Begriff "(R)- und (S)-3-Amino-1-butanol, die am Sauerstoffatom eine Schutzgruppe tragen" bezeichnet die Verbindungen I-R bzw. I-S, in denen R¹ von H verschieden ist.

Im Rahmen der vorliegenden Erfindung steht C₁-C₃-Alkyl für einen Alkylrest mit 1 bis 3 Kohlenstoffatomen, d.h. für Methyl, Ethyl, Propyl oder Isopropyl.

C₁-C₄-Alkyl steht für einen Alkylrest mit 1 bis 4 Kohlenstoffatomen, d.h. für Methyl, Ethyl, Propyl, Isopropyl, n-Butyl, sec-Butyl, Isobutyl oder tert-Butyl.

C₁-C₃-Alkoxy steht für einen C₁-C₃-Alkylrest, der über ein Sauerstoffatom gebunden ist. Beispiele hierfür sind Methoxy, Ethoxy, Propoxy und Isopropoxy.

C₁-C₃-Alkoxy-C₁-C₃-alkyl steht für einen C₁-C₃-Alkylrest, in welchem ein Wasserstoffatom durch eine C₁-C₃-Alkoxygruppe substituiert ist. Beispiele hierfür sind Methoxymethyl, 1- und 2-Methoxyethyl, 1-, 2- und 3-Methoxypropyl, 1- und 2-Methoxyprop-2-yl, Ethoxymethyl, 1- und 2-Ethoxyethyl, 1-, 2- und 3-Ethoxypropyl, 1- und 2-Ethoxyprop-2-yl, Propoxymethyl, 1- und 2-Propoxyethyl, 1-, 2- und 3-Propoxypropyl, 1- und 2-Propoxyprop-2-yl und dergleichen.

Die nachfolgend gemachten Ausführungen zu bevorzugten Ausgestaltungen der erfindungsgemäßen Verfahren, der Reaktanden, Reaktionsbedingungen und der Produkte gelten sowohl für sich genommen als auch insbesondere in Kombination miteinander.

Die Ausführungen gelten dabei für beide erfindungsgemäßen Verfahren, sofern sie überlappen.

Unter im Wesentlichen enantiomerenreinen Verbindungen, insbesondere unter im Wesentlichen enantiomerenreinem (R)-3-Amino-1-butanol, soll im Rahmen der vorliegenden Erfindung verstanden werden, dass diese in einer Enantiomerenreinheit von wenigstens 95 % ee, vorzugsweise wenigstens 96 % ee, besonders bevorzugt wenigstens 98 % ee, stärker bevorzugt wenigstens 99 % ee, noch stärker bevorzugt wenigstens 99,5 % ee, insbesondere wenigstens 99,6 % ee und speziell wenigstens 99,8 % ee vorliegen.

Mit "(R)-Mandelsäure" ist im Wesentlichen enantiomerenreine (R)-Mandelsäure gemeint, d.h. (R)-Mandelsäure in einer Enantiomerenreinheit von wenigstens 95 % ee, vorzugsweise wenigstens 96 % ee, besonders bevorzugt wenigstens 98 % ee, stärker bevorzugt wenigstens 99 % ee, noch stärker bevorzugt wenigstens 99,5 % ee und insbesondere wenigstens 99,6 % ee.

Entsprechend ist mit "(S)-Mandelsäure" im Wesentlichen enantiomerenreine (S)-Mandelsäure gemeint, d.h. (S)-Mandelsäure in einer Enantiomerenreinheit von wenigstens 95 % ee, vorzugsweise wenigstens 96 % ee, besonders bevorzugt wenigstens 98 % ee, stärker bevorzugt wenigstens 99 % ee, noch stärker bevorzugt wenigstens 99,5 % ee und insbesondere wenigstens 99,6 % ee.

Die Einheit "% ee" bezieht sich auf den Enantiomerenüberschuss und ist somit ein Maß für die Enantiomerenreinheit, die auch als optische Reinheit bezeichnet wird. Diese errechnet sich aus der Differenz der molaren Anteile der beiden Enantiomere in einer Enantiomerenmischung. Beispielsweise bedeutet 95 % ee, dass der Anteil des Hauptenantiomers in der Enantiomerenmischung 97,5 % beträgt und der Anteil der dazu spiegelbildlichen Verbindung 2,5 % beträgt.

Bei den Verbindungen I-R und I-S handelt es sich um gegebenenfalls am Sauerstoffatom geschütztes ("O-geschütztes") (R)- bzw. (S)-3-Amino-1-butanol.

Im Enantiomerengemisch, das in Schritt (i) eingesetzt wird, hat R¹ in den Verbindungen I-R und I-S jeweils die gleiche Bedeutung.

In Schritt (i) kann als Enantiomerengemisch das Racemat der Verbindungen I-R und I-S eingesetzt werden; Gemische, in denen eines der Enantiomere angereichert ist, sind aber ebenso geeignet. Da jedoch die übliche Synthese von gegebenenfalls O-geschütztem 3-Amino-1-butanol zu dessen Racemat führt, wird in Schritt (i) häufig das Racemat der Verbindungen I-R und I-S eingesetzt.

Geeignete Schutzgruppen (d.h. geeignete Bedeutungen von R¹, wenn dieses von Wasserstoff verschieden ist) sind C₁-C₄-Alkyl.

R¹ steht daher für Wasserstoff oder C₁-C₄-Alkyl und bevorzugt für Wasserstoff oder tert-Butyl Insbesondere steht R¹ jedoch für Wasserstoff; d.h. in Schritt (i) der erfindungsgemäßen Verfahren wird insbesondere ein Enantiomerengemisch von (R)- und (S)-3-Amino-1-butanol eingesetzt.

In Schritt (i) setzt man (S)- oder (R)-Mandelsäure ein. Man wählt das Mandelsäureenantiomer vorzugsweise so aus, dass es mit der Verbindung I-R das Salz mit dem niedrigsten Löslichkeitsprodukt der in Schritt (i) theoretisch möglichen Salze (insbesondere mit einem niedrigeren Löslichkeitsprodukt als das Salz der Verbindung I-R mit dem entgegengesetzten Mandelsäureantipoden und natürlich auch als das Salz der Verbindung I-S mit dem gewählten Mandelsäureantipoden) im verwendeten Reaktionsmedium bildet. Bei Wahl der unten genannten bevorzugten Lösungsmittel setzt man vorzugsweise (S)-Mandelsäure ein, wenn in den Verbindungen I-R und I-S R¹ für Wasserstoff oder gegebenenfalls substituiertes Benzyl steht. Steht R¹ hingegen für C₁-C₄-Alkyl und insbesondere für tert-Butyl, ist es bevorzugt, in Schritt (i) (R)-Mandelsäure zu verwenden.

Die in Schritt (i) eingesetzte (S)- oder (R)-Mandelsäure wird vorzugsweise in einer Menge von 0,8 bis 1,5 Mol, besonders bevorzugt von 0,8 bis 1,2 Mol, stärker bevorzugt von 0,9 bis 1,1 Mol und noch stärker bevorzugt von 1 bis 1,1 Mol, z.B. 1 bis 1,05 Mol, bezogen auf 1 Mol der Verbindung I-R, die im in Schritt (i) eingesetzten Enantiomerengemisch enthalten ist, eingesetzt. Insbesondere wird (S)- oder (R)-Mandelsäure in etwa äquimolarer Menge, bezogen auf die im Enantiomerengemisch enthaltene Menge an Verbindung I-R, einsetzt. "In etwa äquimolar" soll dabei zum Ausdruck bringen, dass Fehlertoleranzen, die z.B. durch Messungenauigkeiten der Waagen und/oder durch den Reinheitsgrad der Edukte zustande kommen, vom Begriff der Äquimolarität mitumfasst sind.

Ist das in Schritt (i) eingesetzte Enantiomerengemisch das Racemat der Verbindungen I-R und I-S, so wird (S)- oder (R)-Mandelsäure in einer Menge von vorzugsweise 0,4 bis 0,75 Mol, besonders bevorzugt von 0,4 bis 0,6 Mol, stärker bevorzugt von 0,45 bis 0,55 Mol und noch stärker bevorzugt von 0,5 bis 0,55 Mol, z.B. 0,5 bis 0,525 Mol, bezogen auf 1 Mol des Racemats, eingesetzt. Insbesondere wird (S)- oder (R)-Mandelsäure in einer Menge von etwa 0,5 Mol, bezogen auf 1 Mol des Racemats, einsetzt. Bezüglich des Begriffs "etwa" wird auf das oben zu "in etwa äquimolar" Gesagte sinngemäß Bezug genommen.

Die in Schritt (i) eingesetzte, von der (S)- oder (R)-Mandelsäure verschiedene Säure wird vorzugsweise in einer Menge von 0,8 bis 1,5 Mol, besonders bevorzugt von 0,8 bis 1,2 Mol, stärker bevorzugt von 0,9 bis 1,1 Mol und noch stärker bevorzugt von 1 bis 1,1 Mol, z.B. 1 bis 1,05 Mol, bezogen auf 1 Mol der Verbindung I-S, die im in Schritt (i) eingesetzten Enantiomerengemisch enthalten ist, eingesetzt. Insbesondere wird diese Säure in etwa äquimolarer Menge, bezogen auf die im Enantiomerengemisch enthaltene Menge an Verbindung I-S, einsetzt. Bezüglich des Begriffs "in etwa äquimolar" wird auf die obige Ausführung Bezug genommen. Die obigen Angaben zur Säuremenge beziehen sich auf einprotonige Säuren; bei zwei- oder dreiprotonigen Säuren, deren Säurestärke so groß ist, dass auch das zweite und das dritte Proton die Aminogruppe des Edukts protonieren können, gelten entsprechend durch den Faktor 2 bzw. 3 verringerte Mengen.

Ist das in Schritt (i) eingesetzte Enantiomerengemisch das Racemat der Verbindungen I-R und I-S, so wird die von (S)- oder (R)-Mandelsäure verschiedene Säure in einer Menge von 0,4 bis 0,75 Mol, besonders bevorzugt von 0,4 bis 0,6 Mol, stärker bevorzugt von 0,45 bis 0,55 Mol und noch stärker bevorzugt von 0,5 bis 0,55 Mol, z.B. 0,5 bis 0,525 Mol, bezogen auf 1 Mol des Racemats, eingesetzt. Insbesondere wird diese Säure in einer Menge von etwa 0,5 Mol, bezogen auf 1 Mol des Racemats, einsetzt. Bezüglich des Begriffs "etwa" wird auf das oben zu "in etwa äquimolar" Gesagte sinngemäß Bezug genommen. Ebenso wird für den Fall zwei- oder dreiprotoniger Säuren auf das oben Gesagte Bezug genommen.

Die Wahl der von der (S)- oder (R)-Mandelsäure verschiedenen Säure unterliegt keinen speziellen Anforderungen; sie soll jedoch die Salzbildung zwischen der Verbindung I-R und der eingesetzten (S)- bzw. (R)-Mandelsäure nicht negativ beeinflussen; speziell soll diese Säure nicht mit der Verbindung I-R ein Säureadditionssalz mit einem niedrigeren Löslichkeitsprodukt im Reaktionsmedium des Schritts (i) bilden als das Salz der verwendeten (S)- bzw. (R)-Mandelsäure mit der Verbindung I-R. Die Säure soll auch nicht oxidativ wirken. Außerdem soll sie im Reaktionsmedium des Schritts (i) wenigstens teilweise löslich sein und selbstverständlich einen ausreichend hohen pKₛ-Wert besitzen, um die Verbindung I-S unter den Reaktionsbedingungen des Schritts (i) protonieren zu können. Des Weiteren soll sie mit der Verbindung I-S ein unter den Reaktionsbedingungen des Schritts (i) möglichst gut lösliches Salz bilden. Die Säure braucht nicht chiral zu sein; achirale Säuren sind zumindest für das erfindungsgemäße Verfahren, das zur Herstellung von im Wesentlichen enantiomerenreinem (R)-3-Amino-1-butanol dient, völlig ausreichend und aus Kosten- und Zugänglichkeitsgründen bevorzugt. Chirale Säuren sind aber durchaus geeignet; so kann beispielsweise das entgegengesetzte Enantiomer der in Schritt (i) eingesetzten (S)- oder (R)-Mandelsäure eingesetzt werden. Wird in Schritt (i) das Racemat der Verbindungen I-R und I-S verwendet, so kann beispielsweise in Schritt (i) insgesamt racemische Mandelsäure eingesetzt werden. Die Verwendung chiraler Säuren bietet sich beispielsweise dann an, wenn auch im Wesentlichen enantiomerenreines I-S erhalten werden soll.

Vorzugsweise ist die eingesetzte Säure eine einprotonige Säure.

Geeignete achirale Säuren sind z.B. anorganische Säuren, wie Chlorwasserstoff (gasförmig), Salzsäure, Bromwasserstoffsäure oder Schwefelsäure, oder organische Säuren, beispielsweise aliphatische Monocarbonsäuren mit vorzugsweise 1 bis 6 Kohlenstoffatomen, wie Ameisensäure, Essigsäure, Trifluoressigsäure, Propionsäure, Buttersäure, Valeriansäure, Isovaleriansäure und Capronsäure, aromatische Carbonsäuren, wie Benzoesäure, und Sulfonsäuren, wie Methansulfonsäure, Trifluormethansulfonsäure oder Toluolsulfonsäure. Geeignet sind auch Gemische der vorgenannten Säuren. Bevorzugt sind hierunter aliphatische Monocarbonsäuren mit 1 bis 3 Kohlenstoffatomen, wie Ameisensäure, Essigsäure, Trifluoressigsäure und Propionsäure, sowie Gemische davon. Insbesondere verwendet man Essigsäure.

Schritt (i) wird vorzugsweise in einem geeigneten Lösungsmittel durchgeführt. Geeignete Lösungsmittel sind vorzugsweise solche, in denen die Edukte löslich sind, aber - zumindest in einem bestimmten Temperaturbereich - das Salz der eingesetzten (S)-bzw. (R)-Mandelsäure mit der Verbindung I-R nicht oder nicht gut löslich ist, wobei im selben Temperaturbereich das Säureadditionssalz der Verbindung I-S noch ausreichend löslich ist. Als besonders geeignet haben sich C₁-C₄-Alkohole, wie Methanol, Ethanol, Propanol, Isopropanol, n-Butanol, sec-Butanol, Isobutanol und tert-Butanol, und Gemische davon herausgestellt, die gegebenenfalls Wasser in einer Menge von 0,05 bis 10 Gew.-%, bevorzugt 0,1 bis 10 Gew.-%, besonders bevorzugt 0,1 bis 5 Gew.-%, stärker bevorzugt 0,1 bis 1 Gew.-% und insbesondere 0,1 bis 0,5 Gew.-%, bezogen auf das Gesamtgewicht der C₁-C₄-Alkohole und Wasser, enthalten.

Für den Fall, dass R¹ für H steht, ist es bevorzugt, dass die Alkohole 0,05 bis 10 Gew.-%, besonders bevorzugt 0,1 bis 10 Gew.-%, stärker bevorzugt 0,1 bis 5 Gew.-%, noch stärker bevorzugt 0,1 bis 1 Gew.-%, insbesondere 0,1 bis 0,5 Gew.-% und speziell 0,2 bis 0,5 Gew.-% Wasser, bezogen auf das Gesamtgewicht der C₁-C₄-Alkohole und Wasser, enthalten.

Ein besonders bevorzugtes Lösungsmittel ist Isopropanol, das 0,05 bis 10 Gew.-%, vorzugsweise 0,1 bis 10 Gew.-% Wasser, stärker bevorzugt 0,1 bis 5 Gew.-%, noch stärker bevorzugt 0,1 bis 1 Gew.-%, insbesondere 0,1 bis 0,5 Gew.-% und speziell 0,2 bis 0,5 Gew.-% Wasser, bezogen auf das Gesamtgewicht von Isopropanol und Wasser, enthalten kann.

Für den Fall, dass R¹ für H steht, ist ein besonders bevorzugtes Lösungsmittel Isopropanol, das 0,05 bis 10 Gew.-%, vorzugsweise 0,1 bis 10 Gew.-% Wasser, stärker bevorzugt 0,1 bis 5 Gew.-%, noch stärker bevorzugt 0,1 bis 1 Gew.-%, insbesondere 0,1 bis 0,5 Gew.-% und speziell 0,2 bis 0,5 Gew.-% Wasser, bezogen auf das Gesamtgewicht von Isopropanol und Wasser, enthält.

Für den Fall, dass R¹ von Wasserstoff verschieden ist, können die Alkohole auch im Wesentlichen wasserfrei sein, d.h. weniger als 0,05 Gew.-% Wasser, bezogen auf das Gesamtgewicht von Alkohol und Wasser, enthalten.

Die Durchführung des Schritts (i) erfolgt beispielsweise so, dass man das Enantiomerengemisch in einem geeigneten Lösungsmittel vorlegt und mit der (S)- oder (R)-Mandelsäure und der davon verschiedenen Säure gleichzeitig oder nacheinander, auf einmal, portionsweise oder kontinuierlich versetzt. Werden die beiden Säuren nacheinander zugefügt, so ist die Reihenfolge der Zugabe, d.h. ob zuerst die (S)- oder (R)-Mandelsäure und dann die davon verschiedene Säure zugegeben wird oder umgekehrt, nicht von Relevanz. Vorzugsweise werden jedoch beide Säuren nicht auf einmal, sondern kontinuierlich oder portionsweise zugegeben. Das Enantiomerengemisch wird vorzugsweise bei Raumtemperatur vorgelegt; möglich ist es jedoch auch, die Vorlage vor der Zugabe der Säuren zu erwärmen, beispielsweise auf eine Temperatur von >25 °C bis zum Siedepunkt des Lösungsmittels, was allerdings die Reaktionskontrolle erschweren kann, wenn die Salzbildung exotherm erfolgt. Daher wird das Enantiomerengemisch vorzugsweise bei Raumtemperatur vorgelegt.

Durch das Versetzen des Enantiomerengemischs mit der (S)- oder (R)-Mandelsäure und der davon verschiedenen Säure entstehen die Säureadditionssalze der Verbindungen I-R und I-S. Bei Wahl der oben genannten bevorzugten Lösungsmittel sind bei Raumtemperatur in der Regel das (S)- oder (R)-Mandelsäuresalz der Verbindung I-R und gegebenenfalls auch das Säureadditionssalz der Verbindung I-S nicht oder zumindest nicht vollständig löslich und präzipitieren daher im Verlauf ihres Entstehens. Das gewünschte (S)- oder (R)-Mandelsäuresalz der Verbindung I-R muss daher aus dem Präzipitat abgetrennt und isoliert werden.

Bei Wahl der oben genannten bevorzugten Lösungsmittel erfolgt das Abtrennen und Isolieren des (S)- oder (R)-Mandelsäuresalzes der Verbindung I-R vorzugsweise durch dessen Präzipitation und Isolieren des Präzipitats. Da wie gesagt die Säureadditionssalze der Verbindungen I-R und I-S in den oben beschriebenen bevorzugten Lösungsmitteln bei Raumtemperatur (d.h. bei der bevorzugten Temperatur des Schritts (i)) im Allgemeinen nicht oder nicht vollständig löslich sind, werden diese zunächst in Lösung gebracht, was vorzugsweise durch Erwärmen, bevorzugt auf eine Temperatur von 30 °C bis zum Siedepunkt des Gemischs, besonders bevorzugt von 50 °C bis zum Siedepunkt des Gemischs und insbesondere bis zum Siedepunkt des Gemischs, erfolgt. Anschließend wird die Lösung abgekühlt. Das Abkühlen darf nicht zu schnell erfolgen, damit die verschiedenen Säureadditionssalze, die unterschiedliche Löslichkeitsprodukte besitzen, nacheinander und nicht zeitgleich ausfallen. Beim Abkühlen fällt das Säureadditionssalz mit dem geringsten Löslichkeitsprodukt im verwendeten Lösungsmittel zuerst aus. Dies ist in der Regel - zumindest bei Wahl der oben genannten bevorzugten, von der eingesetzten (S)- oder (R)-Mandelsäure verschiedenen Säuren und der oben genannten bevorzugten Lösungsmittel - das (S)- oder (R)-Mandelsäuresalz der Verbindung I-R; dieses wird dann aus dem Gemisch isoliert. Das Isolieren kann mittels bekannter Verfahren, wie Filtrieren, Sedimentieren oder Zentrifugieren erfolgen. Wenn dennoch zuerst das Säureadditionssalz der Verbindung I-S präzipitiert, so wird dieses möglichst vollständig isoliert und anschließend die Lösung weiter abgekühlt, bis das (S)- oder (R)-Mandelsäuresalz der Verbindung I-R präzipitiert, welches dann isoliert wird. Bei Wahl der oben beschriebenen bevorzugten Lösungsmittel S und der oben beschriebenen bevorzugten Säuren, die ja so gewählt sind, dass das Löslichkeitsprodukt ihres Säureadditionssalzes mit der Verbindung I-S in den bevorzugten Lösungsmitteln höher ist als das Löslichkeitsprodukt des (S)- oder (R)-Mandelsäuresalzes der Verbindung I-R, wird jedoch das (S)- oder (R)-Mandelsäuresalz der Verbindung I-R zuerst präzipitieren.

In einer bevorzugten Ausführungsform wird das in Schritt (ii) isolierte (S)- oder (R)-Mandelsäuresalz der Verbindung I-R anschließend ein oder mehrfach, beispielsweise ein-, zwei- oder dreifach, aufgereinigt.

Das Aufreinigen erfolgt vorzugsweise durch ein oder mehrfaches, beispielsweise durch ein-, zwei- oder dreifaches, Umkristallisieren. Für das Umkristallisieren geeignete Lösungsmittel sind die oben genannten C₁-C₄-Alkohole und Gemische davon sowie ihre Gemische mit Wasser, beispielsweise mit Wasser in einer Menge von 0,1 bis 10 Gew.-%, vorzugweise 0,5 bis 5 Gew.-%, besonders bevorzugt 1 bis 5 Gew.-% und insbesondere von 1 bis 3 Gew.-%, bezogen auf das Gesamtgewicht von Alkohol und Wasser. Bevorzugter Alkohol ist auch für das Umkristallisieren Isopropanol und seine Gemische mit Wasser, beispielsweise mit Wasser in einer Menge von 0,1 bis 10 Gew.-%, vorzugweise 0,5 bis 5 Gew.-%, besonders bevorzugt 1 bis 5 Gew.-% und insbesondere von 1 bis 3 Gew.-%, bezogen auf das Gesamtgewicht von Isopropanol und Wasser. Wird mehr als einmal umkristallisiert, so können für die verschiedenen Umkristallisationsschritte unterschiedliche Lösungsmittel oder Lösungsmittelgemische verwendet werden. So kann beispielsweise einmal in einem C₁-C₄-Alkohol, vorzugsweise in Isopropanol, und einmal in einem Alkohol-Wasser-Gemisch, vorzugsweise einem Isopropanol-Wasser-Gemisch mit 0,1 bis 10 Gew.-%, vorzugweise 0,5 bis 5 Gew.-%, besonders bevorzugt 1 bis 5 Gew.-% und insbesondere von 1 bis 3 Gew.-% Wasser, bezogen auf das Gesamtgewicht von isopropanol und Wasser, umkristallisiert werden.

Das Freisetzen der Verbindung I-R aus seinem (S)- oder (R)-Mandelsäuresalz in Schritt (iv) erfolgt vorzugsweise durch Umsetzung des in Schritt (ii) oder (iii) erhaltenen (S)- oder (R)-Mandelsäuresalzes der Verbindung I-R mit einer Base In einem geeigneten Lösungsmittel.

Üblicherweise verwendet man hierzu eine wässrige basische Lösung, beispielsweise wässrige NaOH oder wässrige KOH, und isoliert das freie Amin destillativ oder durch Extraktion in ein organisches Lösungsmittel, wie Diethylether oder Methyl-tert-butylether. Im Falle von (R)-3-Amino-1-butanol (Verbindung I-R, worin R¹ für H steht) ist diese Vorgehensweise jedoch nicht gut geeignet, da das freie (R)-3-Amino-1-butanol einerseits mit Wasser vollständig mischbar ist, so dass eine Extraktion mit or-ganischen Lösungsmitteln nicht oder nur sehr unvollständig gelingt, und andererseits der Siedepunkt von (R)-3-Amino-1-butanol über dem von Wasser liegt, so dass es von den übrigen Umsetzungsprodukten und Verunreinigungen nicht oder nur mit großem Aufwand abgetrennt werden kann.

Insbesondere im Falle des Mandelsäuresalzes von (R)-3-Amino-1-butanol (das ist vorzugsweise das (S)-Mandelsäuresalz) wird das Lösungsmittel daher so gewählt, dass einerseits die Edukte, d.h. das Mandelsäuresalz von (R)-3-Amino-1-butanol und die Base, zumindest teilweise darin löslich sind, und andererseits das freigesetzte (R)-3-Amino-1-butanol sich leicht davon abtrennen lässt. "Teilweise löslich" bedeutet, dass wenigstens 5 Gew.-%, vorzugsweise wenigstens 10 Gew.-% des eingesetzten Salzes und der eingesetzten Base im Lösungsmittel löslich ist.

Erfindungsgemäß ist das Lösungsmittel so gewählt, dass es einen um wenigstens 10 °C, vorzugsweise um wenigstens 20 °C, stärker bevorzugt um wenigstens 50 °C und insbesondere um wenigstens 80 °C höheren Siedepunkt als 3-Amino-1-butanol besitzt.

Lösungsmittel, die den oben genannten Bedingungen genügen, sind beispielsweise organische Amine (dieser Begriff umfasst im Rahmen der vorliegenden Erfindung auch Aminoalkohole) mit einem Schmelzpunkt von vorzugsweise höchstens 50 °C und einem Siedepunkt von vorzugsweise wenigstens 180 °C, besonders bevorzugt wenigstens 200 °C, wie Diethanolamin und Triethanolamin, Di-, Tri- und höhere Polyethylenglykole, Nitrobenzol und Dimethylsulfoxid. Erfindungsgemäß eingesetzte Lösungsmittel sind organische Amine mit einem Schmelzpunkt von vorzugsweise höchstens 50 °C und einem Siedepunkt von vorzugsweise wenigstens 180 °C, besonders bevorzugt wenigstens 200 °C, wie Diethanolamin und Triethanolamin. Speziell verwendet man Triethanolamin.

Geeignete Basen sind in den genannten Lösungsmitteln wenigstens teilweise löslich und besitzen eine ausreichende Basizität, um das (R)-3-Amino-1-butanol aus seinem Mandelsäuresalz freizusetzen. Dementsprechend haben geeignete Basen einen höheren pKₐ-Wert als (R)-3-Amino-1-butanol. Außerdem sollte das aus der Base bei der Freisetzungsreaktion gebildete Produkt (z.B. Methanol bei der Verwendung eines Methanolats) vom freigesetzten (R)-3-Amino-1-butanol leicht abtrennbar sein. Erfindungsgemäß eingesetzte Basen sind Alkalimetall-C₁-C₄-alkoxide, wie Natriummethanolat, Kaliummethanolat, Natriumethanolat, Kaliumethanolat, Natriumpropanolat, Kaliumpropanolat, Natriumisopropanolat, Kaliumisopropanolat, Natrium-n-butanolat, Kalium-n-butanolat, Natrium-tert-butanolat und Kalium-tert-butanolat. Bevorzugte Basen sind Alkalimetall-C₁-C₃-alkoxide, wie Natriummethanolat, Kaliummethanolat, Natriumethanolat, Kallumethanolat, Natriumpropanolat, Kaliumpropanolat, Natriumisopropanolat und Kallumisopropanolat Stärker bevorzugte Basen sind Alkalimetall-C₁-C₂-alkoxide, wie Natriummethanolat, Kaliummethanolat, Natriumethanolat und Kaliumethanolat. Insbesondere verwendet man Natriummethanolat oder Natriumethanolat und speziell Natriummethanolat.

Die Base wird vorzugsweise in wenigstens äquimolaren Mengen in Bezug auf das Mandelsäuresalz von (R)-3-Amino-1-butanol eingesetzt, besonders bevorzugt in einer Menge von 1 bis 2 Mol, stärker bevorzugt von 1 bis 1,5 Mol, noch stärker bevorzugt von 1 bis 1,3 Mol und Insbesondere von 1 bis 1,1 Mol, bezogen auf 1 Mol des Mandelsäuresalzes von (R)-3-Amino-1-butanol.

Vorzugsweise geht man in Schritt (iv) so vor, dass man das Säureadditionssalz im Lösungsmittel vorlegt, gegebenenfalls erwärmt und mit der Base versetzt. Alternativ kann man das Säureadditionssalz Im Lösungsmittel vorlegen, mit der Base versetzen und erst anschließend gegebenenfalls erwärmen. Das Erwärmen erfolgt vorzugsweise auf eine Temperatur von 30 bis 100 °C, besonders bevorzugt von 50 bis 100 °C und insbesondere von 70 bis 90 °C. Anschließend trennt man das freigesetzte (R)-3-Amino-1-butanol aus dem Reaktionsgemisch ab, was vorzugsweise durch (fraktionierte) Destillation, bevorzugt unter verringertem Druck, erfolgt.

Diese Vorgehensweise ist auch für Verbindungen I-R geeignet, in denen R¹ nicht für H steht; hier ist es jedoch auch möglich, als Lösungsmittel Wasser und eine wasserlösliche Base, wie Kalium- oder Natriumhydroxid einzusetzen. Da Verbindungen I-R, in denen R¹ nicht für H steht, mit Wasser im Wesentlichen nicht mischbar sind, können sie beispielsweise durch Extraktion In ein organisches Lösungsmittel, das seinerseits mit Wasser im Wesentlichen nicht mischbar ist, und anschließende Entfernung des organischen Lösungsmittels beispielsweise durch Destillation, gegebenenfalls unter verringertem Druck, isoliert werden. Geeignete organische Lösungsmittel sind beispielsweise aliphatische Kohlenwasserstoffe, wie Pentan, Hexan, Heptan und Octan, cycloaliphatische Kohlenwasserstoffe, wie Cyclohexan und Cyclooctan, Chloralkane, wie Dichlormethan, Chloroform, Tetrachlokohlenstoff, Di- und Trlchlorethan, aromatische Kohlenwasserstoffe, wie Benzol, Toluol und die Xylole, aliphatische Ether, wie Diethylether, Dipropylether, Dibutylether, Methylisobutylether und Methyl-tert-butylether, und Carbonsäureester, wie Ethylacetat, Propylacetat, Ethylpropionat und Propylpropionat. Bevorzugt sind hierunter die genannten aliphatischen Ether und insbesondere Diethylether.

Die Verbindung I-R wird anschließend falls gewünscht und falls erforderlich, d.h. für den Fall, dass R¹ nicht für Wasserstoff steht, zu (R)-3-Amino-1-butanol entschützt.

Hierfür können für die jeweilige Schutzgruppe übliche Entschützungsreaktionen angewendet werden. So kann eine Methylschutzgruppe (R¹ = CH₃) durch Umsetzung mit HCl, HBr, HI, Si(CH₃)₃l oder BBr₃ entfernt werden; höhere Alkylreste (R¹ = C₂-C₄-Alkyl) können durch wasserfreie Trifluoressigsäure oder HBr entfernt werden.

Das erhaltene (R)-3-Amino-1-butanol kann schließlich gewünschtenfalls weiter aufgereinigt werden, was beispielsweise destillativ oder extraktiv erfolgen kann.

Eine besonders bevorzugte Ausführungsform der Erfindung betrifft ein Verfahren zur Trennung eines Enantiomerengemischs von (R)- und (S)-3-Amino-1-butanol, umfassend folgende Schritte:
(i) Umsetzen eines Enantiomerengemischs von (R)- und (S)-3-Amino-1-butanol mit (S)-Mandelsäure und einer von (S)-Mandelsäure verschiedenen Säure;
(ii) Abtrennen und Isolieren des in Schritt (i) gebildeten (S)-Mandelsäuresalzes von (R)-3-Amino-1-butanol;
(iii) gegebenenfalls Reinigen des in Schritt (ii) isolierten (S)-Mandelsäuresalzes von (R)-3-Amino-1-butanol;
(iv) Freisetzen von (R)-3-Amino-1-butanol aus seinem (S)-Mandelsäuresalz; und
(v) gewünschtenfalls Freisetzen von (S)-3-Amino-1-butanol.

Eine besonders bevorzugte Ausführungsform der Erfindung betrifft außerdem ein Verfahren zur Herstellung von im Wesentlichen enantiomerenreinem (R)-3-Amino-1-butanol, umfassend folgende Schritte:
(i) Umsetzen eines Enantiomerengemischs von (R)- und (S)-3-Amino-1-butanol mit (S)-Mandelsäure und einer von (S)-Mandelsäure verschiedenen Säure;
(ii) Abtrennen und Isolieren des in Schritt (i) gebildeten (S)-Mandelsäuresalzes von (R)-3-Amino-1-butanol;
(iii) gegebenenfalls Reinigen des in Schritt (ii) isolierten (S)-Mandelsäuresalzes von (R)-3-Amino-1-butanol; und
(iv) Freisetzen von (R)-3-Amino-1-butano! aus seinem (S)-Mandelsäuresalz.

Bezüglich geeigneter und bevorzugter von (S)-Mandelsäure verschiedener Säuren, den in Schritt (i) eingesetzten Mengen an (S)-Mandelsäure und der davon verschiedenen Säure, der in den einzelnen Schritten verwendeten Lösungsmittel und Reaktanden, wie beispielsweise Basen, und weiterer geeigneter und bevorzugter Reaktionsbedingungen und Verfahrensmaßnahmen wird auf die vorstehenden Ausführungen Bezug genommen.

Eine spezielle Ausführungsform der Erfindung betrifft ein Verfahren zur Herstellung von (R)-3-Amino-1-butanol, umfassend folgende Schritte:
(a) Umsetzen eines Enantiomerengemischs von (R)- und (S)-3-Amino-1-butanol mit (S)-Mandelsäure und Essigsäure in Isopropanol als Lösungsmittel, das 0,1 bis 1 Gew.-% Wasser, bezogen auf das Gesamtgewicht von Isopropanol und Wasser, enthält, wobei (S)-Mandelsäure in einer Menge von 0,1 bis 1,1 Mol, bezogen auf 1 Mol des im Enantiomerengemischs enthaltenen (R)-3-Amino-1-butanols, eingesetzt wird und Essigsäure in einer Menge von 0,1 bis 1,1 Mol, bezogen auf 1 Mol des im Enantiomerengemischs enthaltenen (S)-3-Amino-1-butanols, eingesetzt wird;
(b) Erwärmen des in Schritt (a) erhaltenen Gemischs, bis sich die in Schritt (a) gebildeten und ausgefallenen Säureadditionssalze aufgelöst haben;
(c) Abkühlen der in Schritt (b) erhaltenen Lösung, bis sich ein Präzipitat bildet;
(d) Abtrennen und Isolieren des in Schritt (c) gebildeten Präzipitats;
(e) ein- oder mehrfaches Umkristallisieren des in Schritt (d) isolierten Präzipitats in Isopropanol und/oder Isopropanol, das 0,1 bis 5 Gew.-% Wasser, bezogen auf das Gesamtgewicht von Isopropanol und Wasser, enthält;
(f) Umsetzen des in Schritt (e) erhaltenen (S)-Mandelsäuresalzes von (R)-3-Amino-1-butanol mit einem Alkalimetall-C₁-C₄-alkoxid, vorzugsweise mit Natriummethanolat oder Natriumethanolat und speziell mit Natriummethanolat, in einem organischen Amin mit einem Schmelzpunkt von höchstens 50 °C und einem Siedepunkt von wenigstens 180 °C, vorzugsweise in Diethanolamin oder insbesondere in Triethanolamin bei einer Temperatur von 50 bis 100 °C;
(g) destillative Abtrennung des in Schritt (f) freigesetzten (R)-3-Amino-1-butanols; und
(h) gegebenenfalls weitere Aufreinigung des in Schritt (g) erhaltenen (R)-3-Amino-1-butanols.

Das Aufreinigen in Schritt (h) kann beispielsweise, wie zuvor beschreiben, destillativ oder extraktiv erfolgen.

Soll auch (S)-3-Amino-1-butanol durch die erfindungsgemäßen Verfahren gewonnen werden, so wird in Schritt (ii) das Säureadditionssalz der Verbindung I-S isoliert. Dies erfolgt beispielsweise, wie bereits erwähnt, durch weiteres Abkühlen der Lösung, aus dem bereits präzipitiertes (S)- oder (R)-Mandelsäuresalz der Verbindung I-R isoliert wurde, bis auch das Säureadditionssalz der Verbindung I-S ausfällt. Dieses kann, wie für das (S)- oder (R)-Mandelsäuresalz der Verbindung I-R beschrieben, beispielsweise durch Filtration, Sedimentation oder Zentrifugieren isoliert werden und anschließend analog weiter aufgereinigt werden, beispielsweise durch ein- oder mehrfaches Umkristallisieren. Die Freisetzung der Verbindung I-S aus ihrem Säureadditionssalz und die eventuelle Entschützung zu angereichertem (S)-3-Amino-1-butanol kann analog zur oben beschriebenen Freisetzung und eventuellen Entschützung unter Erhalt von (R)-3-Amino-1-butanol erfolgen.

Um (S)-3-Amino-1-butanol in einer besonders hohen Enantiomerenreinheit zu erhalten, ist es bevorzugt, in Schritt (i) als von (S)- oder (R)-Mandelsäure verschiedene Säure den Antipoden dieser eingesetzten Mandelsäure zu verwenden.

Racemisches 3-Amino-1-butanol ist kommerziell erhältlich oder kann gemäß bekannter Literaturverfahren, wie sie beispielsweise in Chemical Abstracts 1949, 6199 oder in J. Am. Chem. Soc. 1957, 79(14), 3839-3846 beschrieben sind, erhalten werden.

(S)- und (R)-Mandelsäure sind ebenfalls kommerziell erhältlich.

O-geschütztes 3-Amino-1-butanol erhält man durch übliche Verfahren zur Schutzgrup-peneinführung, beispielsweise durch Umsetzung von N-geschütztem 3-Amino-1-butanol mit einem R¹-Halogenid (R¹ ist von H verschieden), wie Methyliodid oder, tert-Butylchlorid, und anschließende Entfernung der N-Schutzgruppe. Als geeignete N-Schutzgruppe bietet sich beispielsweise Boc an, das nach Anbringen der O-Schutzgruppe leicht und selektiv wieder entfernt werden kann, beispielsweise durch saure Hydrolyse.

Die Verwendung von O-Schutzgruppen ist jedoch für die erfindungsgemäßen Trennverfahren nicht notwendig, so dass bevorzugt von Enantiomerengemischen von 3-Amino-1-butanol und Insbesondere von racemischem 3-Amino-1-butanol ausgegangen wird.

Ein weiterer Gegenstand der Erfindung ist das (S)-Mandelsäuresalz der Verbindung I-R, worin R¹ für H steht. Vorzugsweise weist dieses erfindungsgemäße (S)-Mandelsäuresalz eine Enantiomerenreinheit von wenigstens 98 % ee, besonders bevorzugt wenigstens 99 % ee, stärker bevorzugt wenigstens 99,5 % ee, noch stärker bevorzugt wenigstens 99,6 % ee und insbesondere wenigstens 99,8 % ee auf.

Ein weiterer Gegenstand der Erfindung ist das (R)-Mandelsäuresalz der Verbindung I-S, worin R¹ für C₁-C₄-Alkyl und insbesondere tert-Butyl steht. Vorzugsweise weist dieses erfindungsgemäße (R)-Mandelsäuresalz eine Enantiomerenreinheit von wenigstens 98 % ee, besonders bevorzugt wenigstens 99 % ee, stärker bevorzugt wenigstens 99,5 % ee, noch stärker bevorzugt wenigstens 99,6 % ee und Insbesondere wenigstens 99,8 % ee auf.

Durch die erfindungsgemäßen Verfahren erhält man (R)-3-Amino-1-butanol mit einer hohen Enantiomerenreinheit von vorzugsweise wenigstens 98 % ee, besonders bevorzugt wenigstens 99 % ee, stärker bevorzugt von wenigstens 99,5 % ee, noch stärker bevorzugt wenigstens 99,6 % ee und insbesondere wenigstens 99,8 % ee und einer chemischen Reinheit von vorzugsweise wenigstens 98 %, besonders bevorzugt wenigstens 99 % und insbesondere von wenigstens 99,5 % in einer zufriedenstellenden Gesamtausbeute in wenigen, einfachen Reaktionsschritten, die keine komplexen Reaktanden und keine Schutzgruppentechnik erfordern. Gewünschtenfalls erhält man auch das entsprechende S-Enantiomer in einer guten chemischen Reinheit und Enantiomerenreinheit.

Der Enantiomerenüberschuss kann mittels gängiger Verfahren bestimmt werden, beispielsweise durch Bestimmung des optischen Drehwerts oder durch Chromatographie an einer chiralen Phase, beispielsweise durch HPLC oder Gaschromatographie über chirale Säulen.

Die Erfindung wird nun durch die nachfolgenden nichtlimitierenden Beispiele veranschaulicht.

### Beispiele

### 1. Enantiomerentrennung von racemischem 3-Amino-1-butanol

### 1.1 Herstellung des (S)-Mandelsäuresalzes von (R)-3-Amino-1-butanol

Eine Mischung aus racemischem 3-Amino-1-butanol (638 g, 7,16 Mol), Isopropanol (4,3 I) und Wasser (8,6 ml) wurde unter Rühren nacheinander mit (S)-Mandelsäure (544 g, 3,58 Mol) und Essigsäure (214,8 g, 3,58 Mol) versetzt. Das Reaktionsgemisch wurde zum Sieden erhitzt, bis die ausgefallenen Salze komplett aufgelöst waren. Dann ließ man unter Rühren auf 20 °C abkühlen, wobei bei 42 °C eine Präzipitatbildung einsetzte. Die ausgefallenen Kristalle wurden abgesaugt und mit Isopropanol (200 ml) gewaschen. Nach Trocknen an der Luft erhielt man 822 g (47,5 % bezogen auf das eingesetzte Racemat; 95 % bezogen auf das im Racemat enthaltene (R)-3-Amino-1-butanol) (S)-Mandelsäuresalz von (R)-3-Amino-1-butanol mit einer optischen Reinheit von 88,4 % ee im gebundenen Amin.

Das erhaltene (S)-Mandelsäuresalz von (R)-3-Amino-1-butanol wurde in Isopropanol (3 I) suspendiert und das Gemisch wurde zum Sieden erhitzt. Die erhaltene klare Lösung wurde unter Rühren auf 20 °C abgekühlt. Das dabei ausgefallene Salz wurde abgesaugt, mit Isopropanol (100 ml) gewaschen und an der Luft getrocknet. Man erhielt 590 g (S)-Mandelsäuresalz von (R)-3-Amino-1-butanol mit einer optischen Reinheit von 98,5 % ee im gebundenen Amin.

Das aus dieser Umkristallisation erhaltene (S)-Mandelsäuresalz von (R)-3-Amino-1-butanol wurde in Isopropanol (2,4 I) und Wasser (35 ml) suspendiert und das Gemisch wurde zum Sieden erhitzt. Die erhaltene klare Lösung wurde unter Rühren auf 20 °C abgekühlt. Das dabei ausgefallene Salz wurde abgesaugt, mit Isopropanol (100 ml) gewaschen und im Vakuumtrockenschrank getrocknet. Man erhielt 556 g (Gesamtausbeute: 32,2 % bezogen auf das eingesetzte Racemat) (S)-Mandelsäuresalz von (R)-3-Amino-1-butanol mit einer optischen Reinheit von 99,6 % ee im gebundenen Amin.
¹H-NMR (400 MHz, D₂O) δ = 1,30 (d, J = 7 Hz, 3H); 1,75 (mc, 1H); 1,90 (mc, 1 H); 3,45 (mc, 1 H); 1,65-1,80 (m, 2H); 5,00 (s, 1 H); 7,35-7,50 (m, 5H).

| | |
|---|---|
| Schmelzpunkt: | 133-135 °C |
| Drehwert: | [α]_{D} = 77,4° (c = 3,0 in H₂O) |

### 1.2 Freisetzung von (R)-3-Amino-1-butanol aus seinem (S)-Mandelsäuresalz

Das in Beispiel 1.1 erhaltene (S)-Mandelsäuresalz von (R)-3-Amino-1-butanol (372 g, 1,54 Mol) wurde in Triethanolamin (1 l) bei 80 °C suspendiert und die breiige Masse wurde mit Natriummethanolat (277,6 g, 1,54 Mol; 30%ig in Methanol) versetzt, woraufhin eine klare Lösung entstand. Diese wurde auf 100 °C erwärmt und es wurde Vakuum angelegt. Schrittweise wurde ein Druck von 750, 500, 250, 100, 50 und schließlich 20 mbar angelegt. Über eine aufgesetzt Claisenbrücke (kein Rücklauf, keine Kolonne) ging bei einer Kopftemperatur von 50 bis 60 °C ein klares Destillat über, bei dem es sich überwiegend um Methanol handelte. Dann wurde der Druck weiter auf 5 mbar erniedrigt und bei einer Kopftemperatur von 76 °C destillierte (R)-3-Amino-1-butanol über. Das Produkt wurde noch einmal im Wasserstrahlpumpenvakuum destilliert, wobei (R)-3-Amino-1-butanol bei 93 °C und 26 mbar überging. Man erhielt 125 g (91 % d. Th.) (R)-3-Amino-1-butanol als farblose Flüssigkeit mit einer optischen Reinheit von 99,6 % ee.

Die optische Reinheit von (R)-3-Amino-1-butanol wurde über GC bestimmt. Hierzu wurden (R)-3-Amino-1-butanol (200 mg) und Triethylamin (300 mg) in Diethylether (15 ml) gelöst und mit Trifluoressigsäureanhydrid (0,6 ml) versetzt. Nach 30 Minuten Rühren gab man gesättigte Ammoniumchloridlösung (5 ml) zu und rührte weitere 15 Minuten. Anschließend ließ man das Gemisch stehen, bis sich zwei Phasen gebildet hatten, und analysierte eine Probe der oberen klaren Phase per GC.

| | |
|---|---|
| Säule: | Hydrodex-TBDAc, 25 m x 0,25 mm, Macherey & Nagel |
| Inlet-Temperatur: | 250 °C |
| Detektor-Temperatur: | 250 °C |
| Injection-Volumen: | 0,5µl |
| Mode: | Split |
| Split ratio: | 100:1 |
| Carrier gas: | He |
| Flow: | 0,8 ml/min (constant flow) |
| Programm: | |
| Initial temperature: | 135 °C |
| Initial time: | 10 min |
| Rate: | 5 °C/min |
| Final temperature: | 170 °C |
| Final time: | 35 min |
| Retentionszeiten: | |
| R-Enantiomer: | 17,68 min (N-trifluoracyliert) |
| | 21,23 min (N,O-bis-trifluoracyliert) |
| S-Enantiomer: | 18,24 min (N-trifluoracyliert) |
| | 20,11 min (N,O-bis-trifluoracyliert) |

### 2. Enantiomerentrennung von 1-Benzyloxy-3-butylamin

### 2.1 Herstellung des (S)-Mandelsäuresalzes von (R)-1-Benzyloxy-3-butylamin

(S)-Mandelsäure (8,5 g, 56 mmol) und Essigsäure (3,4 g, 56 mmol) wurden in 65 ml Isopropanol bei Raumtemperatur vorgelegt und unter Rühren mit racemischem 1-Benzyloxy-3-butylamin (20 g, 112 mmol) versetzt. Dabei trat eine Temperaturerhöhung auf etwa 40 °C auf. Das Reaktionsgemisch wurde zum Sieden erhitzt, bis die ausgefallenen Salze komplett aufgelöst waren. Dann ließ man über Nacht auf Raumtemperatur abkühlen. Die ausgefallenen Kristalle wurden abgesaugt und mit kaltem Isopropanol (20 ml) gewaschen. Nach Trocknen im Vakuum erhielt man 6,4 g (17,3 % bezogen auf das eingesetzte Racemat; 34,6 % bezogen auf das im Racemat enthaltene (R)-1-Benzyloxy-3-butylamin) (S)-Mandelsäuresalz von (R)-1-Benzyloxy-3-butylamin mit einer optischen Reinheit von 48,4 % ee im gebundenen Amin.

Das erhaltene (S)-Mandelsäuresalz von (R)-1-Benzyloxy-3-butylamin wurde drei Mal in Isopropanol umkristallisiert (30, 20 und 15 ml). Man erhielt 1,8 g (S)-Mandelsäuresalz von (R)-1-Benzyloxy-3-butylamin mit einer optischen Reinheit von 99 % ee im gebundenen Amin.
¹H-NMR (400 MHz, D₂O) δ = 1,25 (d, J = 7 Hz, 3H); 1,75 (mc, 1 H); 1,90 (mc, 1 H); 3,45 (mc, 1 H); 3,70 (m, 2H); 4,55 und 4,60 (AB-System, J_{AB} = 12 Hz, 2H); 5,00 (s, 1 H); 7,35-7,50 (m, 10H).
Schmelzpunkt: 128-130 °C

### 2.2 Freisetzung von (R)-1-Benzyloxy-3-butylamin aus seinem (S)-Mandelsäuresalz

Das in Beispiel 2.1 erhaltene (S)-Mandelsäuresalz von (R)-1-Benzyloxy-3-butylamin wurde in Wasser (10 ml) eingetragen und mit 50%iger NaOH versetzt, bis der pH bei 13 lag. Man extrahierte mit Diethylether (2 x 50 ml), trocknete die organische Phase über Natriumsulfat und entfernte das Lösungsmittel im Ölpumpenvakuum. Man erhielt 0,8 g (9 % bezogen auf das in Beispiel 2.1 eingesetzte Racemat) (R)-1-Benzyloxy-3-butylamin als farbloses Öl mit einer optischen Reinheit von 99 % ee.

### 3. Enantiomerentrennung von 1-tert-Butoxy-3-butylamin

### 3.1 Herstellung des (R)-Mandelsäuresalzes von (R)-1-tert-Butoxy-3-butylamin

(R)-Mandelsäure (15,2 g, 0,1 Mol) und Essigsäure (6,0 g, 0,1 Mol) wurden in 150 ml Isopropanol bei Raumtemperatur vorgelegt und unter Rühren mit racemischem 1-tert-Butoxy-3-butylamin (29 g, 0,2 Mol) versetzt. Dabei trat eine Temperaturerhöhung auf etwa 50 °C auf. Das Reaktionsgemisch wurde zum Sieden erhitzt, bis die ausgefallenen Salze komplett aufgelöst waren. Dann ließ man über Nacht auf Raumtemperatur abkühlen. Die ausgefallenen Kristalle wurden abgesaugt und mit kaltem Isopropanol (20 ml) gewaschen. Nach Trocknen im Vakuum erhielt man 20 g (33,7 % bezogen auf das eingesetzte Racemat; 67,3 % bezogen auf das im Racemat enthaltene (R)-1-tert-Butoxy-3-butylamin) (R)-Mandelsäuresalz von (R)-1-tert-Butoxy-3-butylamin mit einer optischen Reinheit von 70 % ee im gebundenen Amin.

Das erhaltene (R)-Mandelsäuresalz von (R)-1-tert-Butoxy-3-butylamin wurde zwei Mal in Isopropanol umkristallisiert (jeweils 100 ml). Man erhielt 12 g (R)-Mandelsäuresalz von (R)-1-tert-Butoxy-3-butylamin mit einer optischen Reinheit von 99,2 % ee im gebundenen Amin.
¹H-NMR (400 MHz, D₂O) δ = 1,25 (s, 9H); 1,35 (d, J = 7 Hz, 3H); 1,75 (mc, 1 H); 1,90 (mc, 1 H); 3,50 (sext, J = 7Hz, 1 H); 3,65 (mc, 2H); 5,00 (s, 1 H); 7,35-7,50 (m, 5H).

### Schmelzpunkt: 178 °C

### 3.2 Freisetzung von (R)-1-tert-Butoxy-3-butylamin aus seinem (R)-Mandelsäuresalz

Das in Beispiel 3.1 erhaltene (R)-Mandelsäuresalz von (R)-1-tert-Butoxy-3-butylamin wurde in Wasser (50 ml) eingetragen und mit 50%iger NaOH versetzt, bis der pH bei 13 lag. Man extrahierte mit Diethylether (2 x 50 ml), trocknete die organische Phase über Natriumsulfat und entfernte das Lösungsmittel im Wasserstrahlpumpenvakuum. Man erhielt 5,5 g (19 % bezogen auf das in Beispiel 3.1 eingesetzte Racemat) (R)-1-tert-Butoxy-3-butylamin als farbloses Öl mit einer optischen Reinheit von 99,2 % ee.

## Patentansprüche

1. Verfahren zur Trennung eines Enantiomerengemischs von gegebenenfalls am Sauerstoffatom geschütztem (R)- und (S)-3-Amino-1-butanol, umfassend folgende Schritte:
(i) Umsetzen eines Enantiomerengemischs der Verbindungen der Formeln I-R und I-S 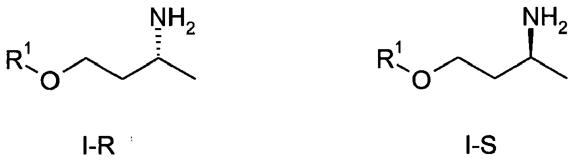
worin R¹ für Wasserstoff oder eine Schutzgruppe, die unter C₁-C₄-Alkyl ausgewählt ist, steht,
mit (S)- oder (R)-Mandelsäure und einer von dieser eingesetzten (S)- oder (R)-Mandelsäure verschiedenen Säure;
(ii) Abtrennen und Isolieren des in Schritt (i) gebildeten (S)- oder (R)-Mandelsäuresalzes der Verbindung der Formel I-R;
(iii) gegebenenfalls Reinigen des in Schritt (ii) isolierten (S)- oder (R)-Mandelsäuresalzes der Verbindung der Formel I-R;
(iv) Freisetzen der Verbindung der Formel I-R aus ihrem (S)- oder (R)-Mandelsäuresalz und gewünschtenfalls Entschützen der Verbindung der Formel I-R, worin R¹ von Wasserstoff verschieden ist, unter Erhalt von (R)-3-Amino-1-butanol; und
(v) gewünschtenfalls Freisetzen der angereicherten Verbindung der Formel I-S und gewünschtenfalls Entschützen der Verbindung der Formel I-S, worin R¹ von Wasserstoff verschieden ist, unter Erhalt von angereichertem (S)-3-Amino-1-butanol,
wobei das Freisetzen der Verbindung der Formel I-R aus ihrem (S)- oder (R)-Mandelsäuresalz in Schritt (iv) durch Umsetzung des in Schritt (ii) oder (iii) erhaltenen (S)- oder (R)-Mandelsäuresalzes der Verbindung der Formel I-R mit einer Base, die unter Alkalimetall-C₁-C₄-alkoxiden ausgewählt ist, in einem Lösungsmittel, das einen um wenigstens 10 °C höheren Siedepunkt als 3-Amino-1-butanol besitzt und unter organischen Aminen mit einem Schmelzpunkt von höchstens 50 °C und einem Siedepunkt von wenigstens 180 °C ausgewählt ist, erfolgt.

2. Verfahren nach Anspruch 1, zur Herstellung von (R)-3-Amino-1-butanol mit einer Enantiomerenreinheit von wenigstens 95 % ee, das gegebenenfalls am Sauerstoffatom eine Schutzgruppe trägt, umfassend folgende Schritte:
(i) Umsetzen eines Enantiomerengemischs der Verbindungen der Formeln I-R und I-S 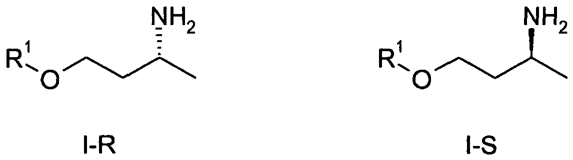
worin R¹ für Wasserstoff oder eine Schutzgruppe, die unter C₁-C₄-Alkyl ausgewählt ist, steht,
mit (S)- oder (R)-Mandelsäure und einer von dieser eingesetzten (S)- oder (R)-Mandelsäure verschiedenen Säure;
(ii) Abtrennen und Isolieren des in Schritt (i) gebildeten (S)- oder (R)-Mandelsäuresalzes der Verbindung der Formel I-R;
(iii) gegebenenfalls Reinigen des in Schritt (ii) isolierten (S)- oder (R)-Mandelsäuresalzes der Verbindung der Formel I-R; und
(iv) Freisetzen der Verbindung der Formel I-R aus ihrem (S)- oder (R)-Mandelsäuresalz und gewünschtenfalls Entschützen der Verbindung der Formel I-R, worin R¹ von Wasserstoff verschieden ist, unter Erhalt von (R)-3-Amino-1-butanol,
wobei das Freisetzen der Verbindung der Formel I-R aus ihrem (S)- oder (R)-Mandelsäuresalz in Schritt (iv) durch Umsetzung des in Schritt (ii) oder (iii) erhaltenen (S)- oder (R)-Mandelsäuresalzes der Verbindung der Formel I-R mit einer Base, die unter Alkalimetall-C₁-C₄-alkoxiden ausgewählt ist, in einem Lösungsmittel, das einen um wenigstens 10 °C höheren Siedepunkt als 3-Amino-1-butanol besitzt und unter organischen Aminen mit einem Schmelzpunkt von höchstens 50 °C und einem Siedepunkt von wenigstens 180 °C ausgewählt ist, erfolgt.

3. Verfahren nach einem der vorhergehenden Ansprüche, wobei R¹ für Wasserstoff steht.

4. Verfahren nach einem der vorhergehenden Ansprüche, wobei man in Schritt (i) (S)-Mandelsäure einsetzt, in Schritt (ii) das (S)-Mandelsäuresalz der Verbindung I-R abtrennt und isoliert, in Schritt (iii) das (S)-Mandelsäuresalz der Verbindung I-R gegebenenfalls reinigt und in Schritt (iv) die Verbindung I-R aus ihrem (S)-Mandelsäuresalz freisetzt.

5. Verfahren nach einem der vorhergehenden Ansprüche, wobei man in Schritt (i) (S)- oder (R)-Mandelsäure in einer Menge von 0,8 bis 1,2 Mol, bezogen auf 1 Mol der im Enantiomerengemisch enthaltenen Verbindung der Formel I-R, einsetzt.

6. Verfahren nach Anspruch 5, wobei man (S)- oder (R)-Mandelsäure in einer Menge von 1 bis 1,05 Mol, bezogen auf 1 Mol der Verbindung der Formel I-R, einsetzt.

7. Verfahren nach einem der vorhergehenden Ansprüche, wobei man in Schritt (i) die von der eingesetzten (S)- oder (R)-Mandelsäure verschiedene Säure in einer Menge von 0,8 bis 1,2 Mol, bezogen auf 1 Mol der im Enantiomerengemisch enthaltenen Verbindung der Formel I-S, einsetzt.

8. Verfahren nach Anspruch 7, wobei man die von der eingesetzten (S)- oder (R)-Mandelsäure verschiedene Säure in einer Menge von 1 bis 1,05 Mol, bezogen auf 1 Mol der Verbindung der Formel I-S, einsetzt.

9. Verfahren nach einem der vorhergehenden Ansprüche, wobei die von (S)- oder (R)-Mandelsäure verschiedene Säure achiral ist.

10. Verfahren nach Anspruch 9, wobei die von (S)- oder (R)-Mandelsäure verschiedene Säure Essigsäure ist.

11. Verfahren nach einem der vorhergehenden Ansprüche, wobei man Schritt (i) in wenigstens einem C₁-C₄-Alkohol, der gegebenenfalls Wasser in einer Menge von 0,1 bis 10 Gew.-%, bezogen auf das Gesamtgewicht des wenigstens einen C₁-C₄-Alkohols und Wasser, enthält, als Lösungsmittel durchführt.

12. Verfahren nach Anspruch 11, wobei man Schritt (i) für den Fall, dass R¹ für Wasserstoff steht, in Isopropanol, das 0,1 bis 10 Gew.-%, vorzugsweise 0,1 bis 5 Gew.-% Wasser, bezogen auf das Gesamtgewicht von Isopropanol und Wasser, enthält, als Lösungsmittel durchführt.

13. Verfahren nach einem der vorhergehenden Ansprüche, wobei das Abtrennen und Isolieren des (S)- oder (R)-Mandelsäuresalzes der Verbindung der Formel I-R durch Präzipitation des (S)- oder (R)-Mandelsäuresalzes der Verbindung der Formel I-R und Isolieren des Präzipitats erfolgt.

14. (S)-Mandelsäuresalz der Verbindung I-R gemäß der Definition in einem der Ansprüche 1 oder 2, worin R¹ für H steht.

15. (R)-Mandelsäuresalz der Verbindung I-S gemäß der Definition in einem der Ansprüche 1 oder 2, worin R¹ für C₁-C₄-Alkyl und vorzugsweise für tert-Butyl steht.

## Claims

1. A process for separating an enantiomer mixture of (R)- and (S)-3-amino-1-butanol optionally protected on the oxygen atom, comprising the following steps:
(i) reacting an enantiomer mixture of the compounds of the formulae I-R and I-S 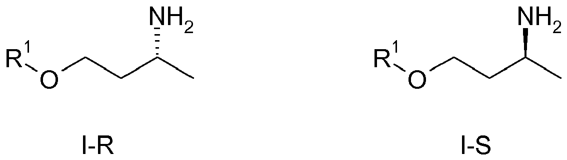 in which R¹ is hydrogen or a protecting group selected from C₁-C₄-alkyl with (S)- or (R)-mandelic acid and an acid different from this (S)- or (R)-mandelic acid used;
(ii) removing and isolating the (S)- or (R)-mandelic salt of the compound of the formula I-R formed in step (i);
(iii) optionally purifying the (S)- or (R)-mandelic salt of the compound of the formula I-R isolated in step (ii);
(iv) releasing the compound of the formula I-R from the (S)- or (R)-mandelic salt thereof and if desired deprotecting the compound of the formula I-R in which R¹ is not hydrogen to obtain (R)-3-amino-1-butanol; and
(v) if desired releasing the enriched compound of the formula I-S and if desired deprotecting the compound of the formula I-S in which R¹ is not hydrogen to obtain enriched (S)-3-amino-1-butanol,
wherein the compound of the formula I-R is released from the (S)- or (R)-mandelic salt thereof in step (iv) by reacting the (S)- or (R)-mandelic salt of the compound of the formula I-R obtained in step (ii) or (iii) with a base selected from alkali metal C₁-C₄-alkoxides in a solvent which possesses a boiling point at least 10°C higher than 3-amino-1-butanol and is selected from organic amines having a melting point of at most 50°C and a boiling point of at least 180°C.

2. The process according to claim 1 for preparing (R)-3-amino-1-butanol having an enantiomeric purity of at least 95% ee which optionally bears a protecting group on the oxygen atom, comprising the following steps:
(i) reacting an enantiomer mixture of the compounds of the formulae I-R and I-S 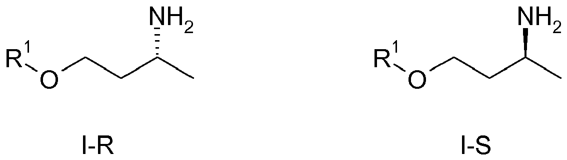 in which R¹ is hydrogen or a protecting group selected from C₁-C₄-alkyl with (S)- or (R)-mandelic acid and an acid different from this (S)- or (R)-mandelic acid used;
(ii) removing and isolating the (S)- or (R)-mandelic salt of the compound of the formula I-R formed in step (i);
(iii) optionally purifying the (S)- or (R)-mandelic salt of the compound of the formula I-R isolated in step (ii); and
(iv) releasing the compound of the formula I-R from the (S)- or (R)-mandelic salt thereof and if desired deprotecting the compound of the formula I-R in which R¹ is not hydrogen to obtain (R)-3-amino-1-butanol
wherein the compound of the formula I-R is released from the (S)- or (R)-mandelic salt thereof in step (iv) by reacting the (S)- or (R)-mandelic salt of the compound of the formula I-R obtained in step (ii) or (iii) with a base selected from alkali metal C₁-C₄-alkoxides in a solvent which possesses a boiling point at least 10°C higher than 3-amino-1-butanol and is selected from organic amines having a melting point of at most 50°C and a boiling point of at least 180°C.

3. The process according to either of the preceding claims, wherein R¹ is hydrogen.

4. The process according to any of the preceding claims, wherein (S)-mandelic acid is used in step (i), the (S)-mandelic salt of the compound I-R is removed and isolated in step (ii), the (S)-mandelic salt of the compound I-R is optionally purified in step (iii), and the compound I-R is released from the (S)-mandelic salt thereof in step (iv).

5. The process according to any of the preceding claims, wherein (S)- or (R)-mandelic acid is used in step (i) in an amount of 0.8 to 1.2 mol, based on 1 mol of the compound of the formula I-R present in the enantiomer mixture.

6. The process according to claim 5, wherein (S)- or (R)-mandelic acid is used in an amount of 1 to 1.05 mol, based on 1 mol of the compound of the formula I-R.

7. The process according to any of the preceding claims, wherein the acid different from the (S)- or (R)-mandelic acid used is used in step (i) in an amount of 0.8 to 1.2 mol, based on 1 mol of the compound of the formula I-S present in the enantiomer mixture.

8. The process according to claim 7, wherein the acid different from the (S)- or (R)-mandelic acid used is used in an amount of 1 to 1.05 mol, based on 1 mol of the compound of the formula I-S.

9. The process according to any of the preceding claims, wherein the acid different from (S)- or (R)-mandelic acid is achiral.

10. The process according to claim 9, wherein the acid different from (S)- or (R)-mandelic acid is acetic acid.

11. The process according to any of the preceding claims, wherein step (i) is performed in the presence of at least one C₁-C₄-alcohol which optionally comprises water in an amount of 0.1 to 10% by weight, based on the total weight of the at least one C₁-C₄-alcohol and water, as a solvent.

12. The process according to claim 11, wherein step (i), in the case that R¹ is hydrogen, is performed in isopropanol which comprises 0.1 to 10% by weight, preferably 0.1 to 5% by weight, of water, based on the total weight of isopropanol and water, as a solvent.

13. The process according to any of the preceding claims, wherein the (S)- or (R)-mandelic salt of the compound of the formula I-R is removed and isolated by precipitation of the (S)- or (R)-mandelic salt of the compound of the formula I-R and isolation of the precipitate.

14. An (S)-mandelic salt of the compound I-R as defined in either of claims 1 and 2, in which R¹ is H.

15. An (R)-mandelic salt of the compound I-S as defined in either of claims 1 and 2, in which R¹ is C₁-C₄-alkyl and preferably tert-butyl.

## Revendications

1. Procédé de séparation d'un mélange d'énantiomères de (R)- et de (S)-3-amino-1-butanol éventuellement protégés sur l'atome d'oxygène, comprenant les étapes suivantes :
(i) la mise en réaction d'un mélange d'énantiomères des composés de formules I-R et I-S 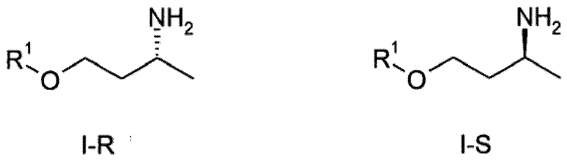
dans lesquelles R¹ représente l'hydrogène ou un groupe protecteur choisi parmi alkyle en C₁-C₄,
avec de l'acide (S)- ou (R)-mandélique et un acide différent de cet acide (S)- ou (R)-mandélique utilisé,
(ii) la séparation et l'isolement du sel de l'acide (S)- ou (R)-mandélique du composé de formule I-R formé à l'étape (i) ;
(iii) éventuellement la purification du sel de l' acide (S)- ou (R)-mandélique du composé de formule I-R isolé à l'étape (ii) ;
(iv) la libération du composé de formule I-R de son sel de l'acide (S)- ou (R)-mandélique et, si on le souhaite, la déprotection du composé de formule I-R dans lequel R¹ est différent de l'hydrogène pour obtenir le (R)-3-amino-1-butanol ; et
(v) si on le souhaite, la libération du composé de formule I-S enrichi et, si on le souhaite, la déprotection du composé de formule I-S dans lequel R¹ est différent de l'hydrogène pour obtenir le (S)-3-amino-1-butanol enrichi ;
la libération du composé de formule I-R de son sel de l'acide (S)- ou (R)-mandélique à l'étape (iv) ayant lieu par mise en réaction du sel de l'acide (S)- ou (R)-mandélique du composé de formule I-R obtenu à l'étape (ii) ou (iii) avec une base, choisie parmi les alcoxydes en C₁-C₄ de métaux alcalins, dans un solvant, qui présente un poids d'ébullition supérieur d'au moins 10 °C au 3-amino-1-butanol et qui est choisi parmi les amines organiques ayant un point de fusion d'au plus 50 °C et un point d'ébullition d'au moins 180 °C.

2. Procédé selon la revendication 1, pour la fabrication de (R)-3-amino-1-butanol ayant une pureté énantiomérique d'au moins 95 % ee, qui porte éventuellement un groupe protecteur sur l'atome d'oxygène, comprenant les étapes suivantes :
(i) la mise en réaction d'un mélange d'énantiomères des composés de formules I-R et I-S 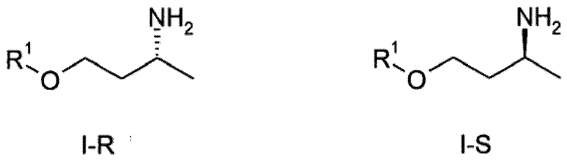
dans lesquelles R¹ représente l'hydrogène ou un groupe protecteur choisi parmi alkyle en C₁-C₄,
avec de l'acide (S)- ou (R)-mandélique et un acide différent de cet acide (S)- ou (R)-mandélique utilisé ;
(ii) la séparation et l'isolement du sel de l'acide (S)- ou (R)-mandélique du composé de formule I-R formé à l'étape (i) ;
(iii) éventuellement la purification du sel de l'acide (S)- ou (R)-mandélique du composé de formule I-R isolé à l'étape (ii) ; et
(iv) la libération du composé de formule I-R de son sel de l'acide (S)- ou (R)-mandélique et, si on le souhaite, la déprotection du composé de formule I-R dans lequel R¹ est différent de l'hydrogène pour obtenir le (R)-3-amino-1-butanol,
la libération du composé de formule I-R de son sel de l'acide (S)- ou (R)-mandélique à l'étape (iv) ayant lieu par mise en réaction du sel de l'acide (S)- ou (R)-mandélique du composé de formule I-R obtenu à l'étape (ii) ou (iii) avec une base, choisie parmi les alcoxydes en C₁-C₄ de métaux alcalins, dans un solvant, qui présente un poids d'ébullition supérieur d'au moins 10 °C au 3-amino-1-butanol et qui est choisi parmi les amines organiques ayant un point de fusion d'au plus 50 °C et un point d'ébullition d'au moins 180 °C.

3. Procédé selon l'une quelconque des revendications précédentes, dans lequel R¹ représente l'hydrogène.

4. Procédé selon l'une quelconque des revendications précédentes, dans lequel de l'acide (S)-mandélique est utilisé à l'étape (i), le sel de l'acide (S)-mandélique du composé I-R est séparé et isolé à l'étape (ii), le sel de l'acide (S)-mandélique du composé I-R est éventuellement purifié à l'étape (iii), et le composé I-R est libéré de son sel de l'acide (S)-mandélique à l'étape (iv).

5. Procédé selon l'une quelconque des revendications précédentes, dans lequel l'acide (S)- ou (R)-mandélique est utilisé à l'étape (i) en une quantité de 0,8 à 1,2 mol, par rapport à 1 mol du composé de formule I-R contenu dans le mélange d'énantiomères.

6. Procédé selon la revendication 5, dans lequel l'acide (S)- ou (R)-mandélique est utilisé en une quantité de 1 à 1,05 mol, par rapport à 1 mol du composé de formule I-R.

7. Procédé selon l'une quelconque des revendications précédentes, dans lequel l'acide différent de l'acide (S)- ou (R)-mandélique utilisé à l'étape (i) est utilisé en une quantité de 0,8 à 1,2 mol, par rapport à 1 mol du composé de formule I-S contenu dans le mélange d'énantiomères.

8. Procédé selon la revendication 7, dans lequel l'acide différent de l'acide (S)- ou (R)-mandélique utilisé est utilisé en une quantité de 1 à 1,05 mol, par rapport à 1 mol du composé de formule I-S.

9. Procédé selon l'une quelconque des revendications précédentes, dans lequel l'acide différent de l'acide (S)- ou (R)-mandélique est achiral.

10. Procédé selon la revendication 9, dans lequel l'acide différent de l'acide (S)- ou (R)-mandélique est l'acide acétique.

11. Procédé selon l'une quelconque des revendications précédentes, dans lequel l'étape (i) est réalisée dans au moins un alcool en C₁-C₄, qui contient éventuellement de l'eau en une quantité de 0,1 à 10 % en poids, par rapport au poids total du ou des alcools en C₁-C₄ et de l'eau, en tant que solvant.

12. Procédé selon la revendication 11, dans lequel l'étape (i) est, lorsque R¹ représente l'hydrogène, réalisée dans de l'isopropanol qui contient 0,1 à 10 % en poids, de préférence 0,1 à 5 % en poids, d'eau, par rapport au poids total de l'isopropanol et de l'eau, en tant que solvant.

13. Procédé selon l'une quelconque des revendications précédentes, dans lequel la séparation et l'isolement du sel de l'acide (S)- ou (R)-mandélique du composé de formule I-R ont lieu par précipitation du sel de l'acide (S)- ou (R)-mandélique du composé de formule I-R et isolement du précipité.

14. Sel de l' acide (S)-mandélique du composé I-R selon la définition dans l'une quelconque des revendications 1 ou 2, dans lequel R¹ représente H.

15. Sel de l'acide (R)-mandélique du composé I-S selon la définition dans l'une quelconque des revendications 1 ou 2, dans lequel R¹ représente alkyle en C₁-C₄ et de préférence tert-butyle.
